# FASCICULE DE BREVET EUROPEEN

(11) **EP 1 787 585 B1**
(45) Date de publication et mention de la délivrance du brevet: **11.06.2008**
(21) Numéro de dépôt: 05356199.9
(22) Date de dépôt: 15.11.2005
(51) Int. Cl.: A61B 6/02, H05G 1/04, H04N 3/00, A61B 6/14, H05G 1/64, A61B 6/03, A61B 6/00

(54) **Procédé d'asservissement d'une source de rayons X d'un dispositif de radiographie**
X-ray source with a feedback loop in a radiography apparatus
Röntgenstrahlquelle mit Rückkoppelungskreis in einem Röntgenapparat

(43) Date de publication de la demande: 23.05.2007
(73) Titulaire: Trophy, 77437 Croissy Beaubourg (FR)
(72) Inventeur: Inglese, Jean-Marc, 77437 Marne-la-Vallee Cedex 2 (FR); Bothorel, Sylvie Marie Gisèle, 77437 Marne-la-Vallee Cedex 2 (FR); Lemaire, Alain Paul, 77437 Marne-la-Vallee Cedex 2 (FR); Boucly, Alain, 77437 Marne-la-Vallee Cedex 2 (FR)
(74) Mandataire: Petit, Maxime

(56) Documents cités:
- EP-A- 1 426 009
- US-A- 4 030 119
- US-A- 5 018 177
- US-A1- 2003 169 850
- US-B1- 6 246 745
- US-B1- 6 377 656

## Description

### Domaine technique

La présente invention concerne un procédé d'asservissement d'une source de rayons X d'un dispositif de radiographie numérique et un équipement de radiographie numérique utilisant une source asservie. L'invention trouve des applications dans le domaine de la radiographie médicale, et en particulier dans celui de la radiographie dentaire.

### Etat de la technique antérieure

Les dispositifs de radiographie numérique présentent un capteur capable de générer des signaux électriques qui sont fonction de l'intensité du rayonnement et plus précisément de l'énergie reçue en tout point d'une surface sensible. A cet effet la surface sensible du capteur est subdivisée en une matrice d'éléments sensibles qui, par analogie avec l'image susceptible d'être formée à partir du signal du capteur, sont encore désignés par « pixels ». La surface sensible est associée à un scintillateur qui convertit le rayonnement X reçu par le capteur en un rayonnement dont la longueur d'onde est compatible avec un spectre de sensibilité des pixels.

Deux technologies distinctes sont utilisées pour constituer les pixels du capteur. La première dite CCD (Charge Coupled Device), fonctionne selon un principe d'accumulation de charges, en réponse au rayonnement reçu. Lors de la lecture, séquentielle, les charges sont transférées de proche en proche d'un pixel au pixel suivant d'une ligne ou d'une colonne de pixels de la matrice. Le transfert des charges est cadencée au moyen de registres à décalage pilotés par une horloge. Le transfert des charges provoque également le rafraîchissement des pixels, qui sont entièrement déchargés à leur lecture.

Une deuxième technologie est dite MOS (Metal Oxide Semi-conductor) ou CMOS (Complementary Metal Oxide Semiconductor). Elle utilise des transistors à effet de champ à grille isolée. Chaque pixel présente une capacité interne, chargée (respectivement déchargée) lors d'une étape de rafraîchissement, et une diode photosensible qui, sous l'effet du rayonnement, décharge (respectivement charge) peu à peu la capacité. Lors de la lecture, on vient mesurer la tension aux bornes de la capacité qui décroît (respectivement croît) proportionnellement à la quantité de rayonnement reçue pendant une période d'intégration s'écoulant depuis le dernier rafraîchissement. Les pixels de type CMOS peuvent présenter des commandes distinctes pour provoquer leur lecture et leur rafraîchissement. Ainsi, la simple lecture n'implique pas nécessairement le rafraîchissement.

Bien qu'une lecture séquentielle ne soit pas requise pour des pixels de type CMOS, elle est néanmoins retenue dans un certain nombre de dispositifs, à l'instar des pixels CCD. La lecture séquentielle présente l'avantage de fournir le signal de lecture en utilisant un nombre réduit de conducteurs électriques et donc de connexions électriques. Un nombre réduit de conducteurs et de connecteurs présente un avantage en particulier pour les capteurs intra-oraux, c'est à dire les capteurs spécifiques de la radiographie dentaire.

La lecture séquentielle et le rafraîchissement des pixels CMOS est illustrée, par exemple, par les documents (1) à (4) auxquels on pourra se référer, bien qu'ils ne relèvent pas du domaine de la radiographie.

Le document US-A-4 030 199 décrit un appareil comme défini dans le préambule de la revendication 10.

Une des difficultés, qui existe quel que soit le type de pixels du capteur, est de contrôler la source de rayons X de manière à éviter à la fois une surexposition et une sous exposition des pixels. Le contrôle de la source de rayons X est également importante pour éviter au patient radiographié d'être soumis à une dose d'énergie supérieure à celle strictement nécessaire à la production d'une image.
A cet effet, il est connu de placer au voisinage de la surface sensible du capteur un ou plusieurs capteurs de dose dont la fonction est de mesurer l'énergie radiative reçue et de provoquer la coupure de la source lorsqu'une dose prédéterminée est atteinte. Les capteurs de dose sont cependant incapables de rendre compte de l'état de toutes les parties de l'image, et n'empêchent pas l'existence de zones locales sur- ou sous exposées. Il suffit en effet qu'un capteur de dose soit situé en face d'une partie particulièrement dense ou particulièrement transparente de tissus radiographiés pour ne pas rendre compte correctement de la dose reçue par les autres tissus. Tel est le cas, par exemple, d'un capteur de dose situé dans l'alignement d'un amalgame dentaire et de la source de rayons X.

Une exposition inappropriée par rapport à la latitude d'exposition nominale du capteur provoque soit une dégradation importante du rapport de signal sur bruit, soit une saturation des pixels, et nuit, dans les deux cas, à la qualité de l'image radiographique.

### Exposé de l'invention

L'invention a pour but de proposer un procédé d'asservissement d'une source de rayons X et un dispositif de radiographie ne présentant pas les difficultés mentionnées ci-dessus.

Un but est en particulier de contrôler avec précision la dose de rayonnement reçue, tout en évitant des surexpositions ou des sous-expositions locales de l'image radiographique obtenue.

Un but est encore de contrôler la source de manière à optimiser l'exposition pour un certain type de tissus.

Un autre but est de limiter la dose de rayonnement reçue par le patient sans porter préjudice à la qualité des images radiographiques.

Pour atteindre ces buts, l'invention a plus précisément pour objet un procédé d'asservissement d'une source de rayons X dans un dispositif de radiographie comprenant un capteur d'image du type MOS avec des pixels pourvus respectivement d'une commande de lecture et d'une commande de rafraîchissement indépendantes, dans lequel, lors d'une opération de radiographie on sollicite de manière répétée les commandes de lecture d'une pluralité de pixels du capteur d'image tout en maintenant une émission de rayons X, de manière a établir, en réponse à chaque lecture au moins une caractéristique d'acquisition d'image, et dans lequel on interrompt l'émission de rayons X lorsque la caractéristique d'acquisition d'image correspond à une caractéristique d'acquisition d'image de consigne.

L'asservissement de la source, c'est à dire le maintien ou l'interruption de l'émission de rayons X a lieu de préférence en maintenant ou en interrompant l'alimentation en énergie de la source de rayons X. D'autres modes d'asservissement, utilisant un obturateur permettent également d'interrompre ou non l'émission des rayons.

Grâce à l'utilisation de pixels du type MOS, c'est à dire des pixels MOS ou CMOS à grille isolée, et en particulier des pixels à commande de lecture indépendante, il est possible d'établir et de suivre l'évolution de la caractéristique d'image sans perturber l'intégration de l'image par les pixels. Le rafraîchissement n'est en effet provoqué que lorsqu'une image de qualité suffisante est disponible, c'est-à-dire après que la caractéristique d'acquisition de consigne soit atteinte.

La caractéristique retenue pour interrompre l'émission de rayons X, n'est plus liée à un capteur de dose, ni même à plusieurs capteurs de dose, mais est établi à partir de la lecture d'un nombre de pixels du capteur qui serait suffisant pour établir une image lisible. Il est possible, de manière optimale, d'utiliser le signal de lecture de tous les pixels du capteur pour établir la caractéristiques d'image. Il est aussi possible d'utiliser le signal d'un sous-ensemble de pixels répartis, de préférence de manière uniforme à la surface sensible du capteur. Ceci revient à utiliser une image sous échantillonnée pour établir la caractéristique d'acquisition d'image, ou, en d'autres termes, d'établir la caractéristique d'acquisition pour une image sous échantillonnée représentant l'image susceptible d'être produite par le capteur.

Différentes caractéristiques d'acquisition d'image peuvent être établies et retenues pour l'asservissement de la source. Il s'agit, par exemple, d'un nombre de pixels, pris parmi l'ensemble des pixels du capteur, ou un sous ensemble de pixels, correspondant à au moins une valeur de densité d'image, c'est- à-dire dont le signal est inférieur à une valeur de consigne.

Selon une autre possibilité, la caractéristique d'acquisition est un étalement du spectre de densité des pixels. La caractéristique de consigne est alors une valeur d'étalement de seuil.

Selon encore une autre possibilité, la caractéristique d'acquisition est un histogramme. L'histogramme, susceptible d'être représenté graphiquement, est compris comme une relation entre des densités, c'est-à-dire des valeurs de gris de l'image susceptible d'être formée à un instant donné à partir du signal de lecture, et le nombre de pixels de l'image ayant respectivement ces densités.

La caractéristique de consigne retenue est alors une caractéristique particulière de tout ou partie de l'histogramme. En particulier la caractéristique de consigne peut être une forme d'histogramme particulière ou une forme d'histogramme centrée sur au moins l'un parmi des pics caractéristiques des tissus mous, des os et de la dentine, de l'émail et/ou des matières opaques.

Par matières opaques en entend des matériaux tels que les amalgames ou les parties métalliques susceptibles de se trouver dans la bouche du patient.

Lorsque la caractéristique d'acquisition correspond à la caractéristique de consigne, on effectue la lecture des pixels du capteur, de manière à fournir un signal de lecture utilisable pour la formation d'une image. Dès que le signal de lecture est disponible, on peut solliciter la commande de rafraîchissement des pixels.

L'invention concerne également un dispositif de radiographie comprenant une source de rayons X et un capteur d'image du type MOS avec des pixels pourvus respectivement d'une commande de lecture et d'une commande de rafraîchissement indépendantes, un séquenceur pour appliquer aux commandes de lecture une série de signaux de sollicitation, répartis sur une période d'exposition radiologique, et un calculateur. Le calculateur reçoit des signaux de lecture fournis par les pixels en réponse aux signaux de sollicitation, pour établir une caractéristique d'acquisition d'image et pour comparer la caractéristique d'acquisition à une caractéristique d'acquisition de consigne. Enfin, un moyen d'interruption de l'exposition radiologique, piloté par le calculateur est prévu, pour interrompre l'exposition lorsque la caractéristique d'acquisition d'image correspond à la caractéristique d'acquisition de consigne.

Comme indiqué précédemment, le moyen pour interrompre l'exposition peut agir sur l'alimentation de la source de rayon X et/ou sur la propagation du faisceau. Il s'agit, par exemple, d'un simple interrupteur commandé connecté en série dans le circuit d'alimentation électrique de la source de rayons X.

Le dispositif peut comporter en outre un moyen de restitution d'image pour produire une image à partir des signaux de lecture fournis par les pixels. Il s'agit par exemple d'un ordinateur ou d'une unité de traitement des signaux pourvue d'un moniteur ou d'une imprimante, telle qu'une imprimante à laser.

D'autres caractéristiques et avantages de l'invention ressortiront de la description qui va suivre, en référence aux figures des dessins annexés. Cette description est donnée à titre purement illustratif et non limitatif.

### Brève description des dessins

La figure 1 est un schéma électrique d'un pixel individuel de type CMOS à double commande susceptible d'être utilisé dans un dispositif conforme à l'invention
La figure 2 est une représentation schématique d'une matrice de pixels utilisant des pixels conformes à la figure 1.
La figure 3 est un organigramme décrivant une série d'étapes de la mise en oeuvre d'un procédé conforme à l'invention
La figure 4 est un chronogramme simplifié illustrant un aspect du fonctionnement d'un dispositif conforme à l'invention.
Les figures 5 à 7 sont des histogrammes illustrant différents stades de l'acquisition d'une image radiographique.
La figure 8 est une représentation simplifiée et schématique d'un dispositif conforme à l'invention.

### Description détaillée de modes de mise en oeuvre de l'invention

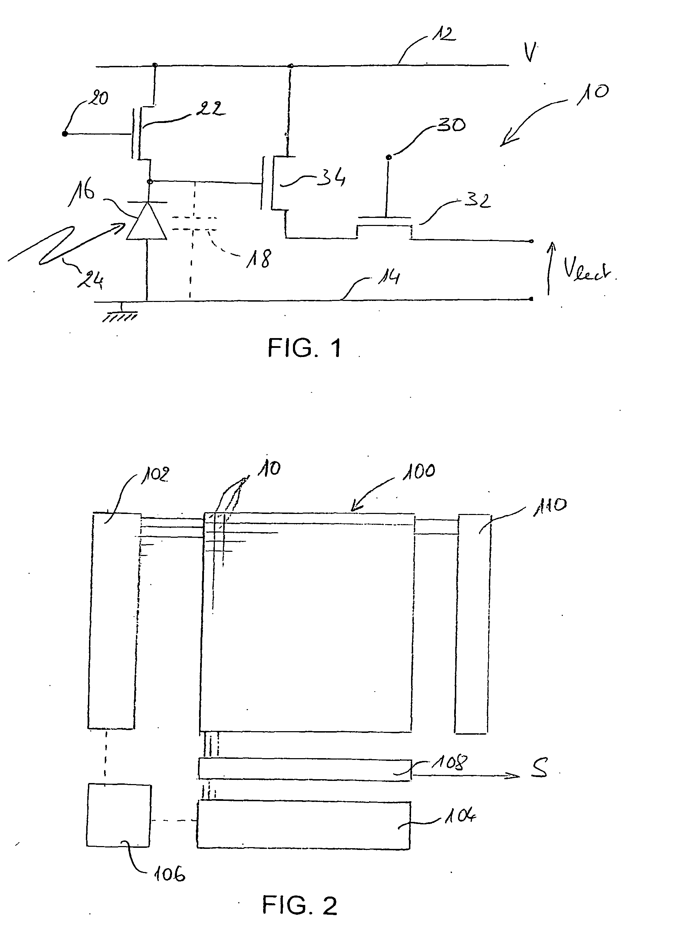

La figure 1 montre un schéma équivalent d'un pixel de type MOS/CMOS 10 alimenté entre une ligne d'alimentation 12 et une ligne de référence 14. Le pixel est construit autour d'une diode 16 sensible à la lumière et d'un condensateur 18 connecté en parallèle à la diode. Le condensateur est représenté en trait discontinu, dans la mesure ou il s'agit non pas d'un composant autonome mais d'une capacité inhérente à la structure MOS/CMOS.

La référence 20 indique une borne servant de commande de rafraîchissement du pixel. Lorsqu'une tension de rafraîchissement est appliquée à la borne 20, un transistor 22 relié à la ligne d'alimentation 12 vient charger le condensateur 18, en portant son potentiel à une valeur de charge V voisine de la tension d'alimentation.

En l'absence de lumière reçue par le pixel, la tension de charge V est conservée. En revanche, en réponse à un rayonnement lumineux 24, un courant inverse de la diode 16 vient décharger progressivement le condensateur 18. La tension aux bornes du condensateur 18 diminue ainsi en fonction de l'intensité lumineuse reçue et en fonction de la durée d'exposition. Il convient de noter à ce sujet que le rayonnement lumineux 24 reçu par le pixel n'est pas un rayonnement X mais un rayonnement lumineux visible obtenu par conversion du rayonnement X par un scintillateur intégré au capteur d'images.

La référence 30 indique une borne servant de commande de lecture du pixel. Lorsqu'une impulsion d'adressage appropriée est appliquée à cette borne, un transistor de lecture 32, associé à un transistor suiveur 34 délivre une tension de lecture V_{lect} représentative de la tension de charge résiduelle aux bornes du condensateur 18. Cette tension de lecture peut être utilisée comme signal de lecture du pixel et être fournie par le capteur à une unité de visualisation d'image.

Grâce au transistor suiveur 34, dont la grille isolée est reliée au condensateur 18, la lecture est sans influence sur l'état de charge du condensateur 18, et sur la tension à ses bornes. La lecture ne perturbe donc pas l'intégration du signal lumineux reçu.

A titre de variante, on peut utiliser un pixel sensiblement identique, mais dans lequel le courant inverse de la diode vient non pas décharger mais charger le condensateur. Dans ce cas le rafraîchissement consiste à décharger le condensateur et le rayonnement reçu tend à faire croître la tension aux bornes du condensateur. Par simplification, la description qui suit est donnée pour des pixels dont la tension décroît avec l'énergie reçue. Elle s'applique toutefois, mutatis mutandis, pour des pixels correspondant à la variante indiquée ici.

Le pixel de la figure 1 est intégré à une matrice de pixels 100 du capteur d'image. Les pixels 10 sont arrangés en lignes et en colonnes comme le montre la figure 2.

Un signal de lecture du capteur d'image est obtenu en adressant séquentiellement les pixels du capteur au moyen de registres à décalage de ligne et de colonne. Les registres sont indiqués avec les références 102 et 104. Ils sont cadencés par une horloge ou un séquenceur 106. Associés à des amplificateurs de colonne 108, ils délivrent un signal de lecture séquentiel S. S'agissant de composants MOS/CMOS, une lecture simultanée en parallèle des pixels est également possible. La lecture séquentielle, comparable à la lecture de pixels de type CCD, présente toutefois la particularité d'autoriser un transfert du signal sur un faible nombre de conducteurs électriques. Le signal peut être transmis, par exemple, sur un câble bifilaire. Cet aspect constitue un avantage pour des capteurs intra-oraux pour lesquels les impératifs de connexion et d'hygiène sont souvent difficiles à concilier.

Le registre à décalage des lignes 102 permet de fournir des impulsions qui appliquées aux commandes de lecture permettent de lire la tension des pixels. Une unité 110, associée éventuellement au registre 102, est prévue pour fournir les impulsions de rafraîchissement aux bornes 20 de chaque pixel.

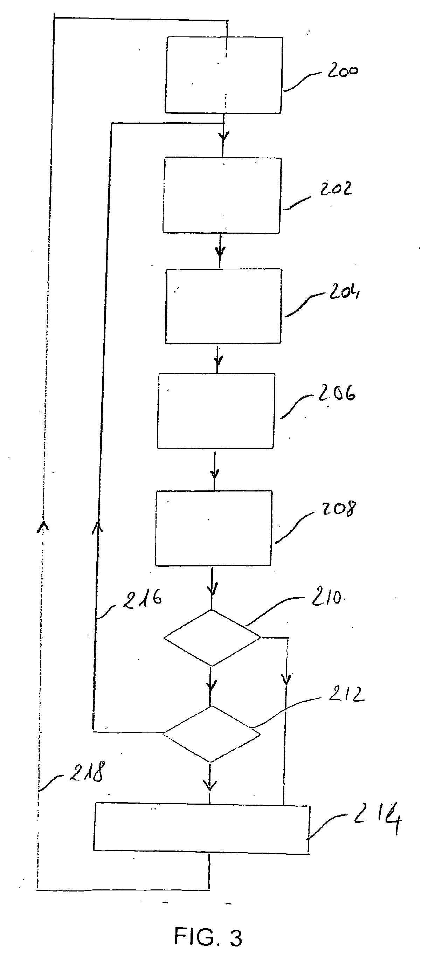

Une illustration du fonctionnement d'un dispositif conforme à l'invention est donnée par l'organigramme de la figure 3, en combinaison avec la description qui précède.

Une première étape 200 consiste à rafraîchir tous les pixels du capteur. Le rafraîchissement peut être séquentiel ou simultané pour l'ensemble des pixels ou pour un sous ensemble de pixels. Vient ensuite une phase d'acquisition 202 lors de laquelle une source de rayons X du dispositif de radiographie est alimentée. Les pixels intègrent la lumière et la tension aux bornes des condensateurs respectifs diminue en fonction de l'énergie reçue.

L'acquisition est suivie par une étape de lecture 204 lors de laquelle un signal de lecture est obtenu en appliquant une impulsion de lecture aux bornes de commande de tous les pixels ou d'un sous ensemble de pixels du capteur. Il s'agit de préférence d'un sous-ensemble de pixels susceptibles de fournir une image sous-échantillonnée.

La lecture 204 est suivie d'une étape de calcul 206 consistant à établir une caractéristique d'acquisition d'image, c'est à dire une caractéristique qu'aurait une image produite avec les pixels à ce stade de l'acquisition. La caractéristique d'acquisition est, par exemple, un histogramme, ou une autre caractéristique mentionnée précédemment.

Dès que la caractéristique d'acquisition est établie, elle est comparée à une caractéristique de consigne. Il s'agit, par exemple, de comparer le nombre de pixels associés à un jeu de valeurs de densité identiques. Lorsque la caractéristique d'acquisition est un histogramme, la comparaison peut notamment porter sur la largeur de son étalement, sur son enveloppe, sa surface, ou encore sur la présence ou l'emplacement de pics caractéristiques. Elle peut aussi consister en un simple calcul d'inter-corrélation d'un histogramme et l'histogramme de consigne.

Le résultat de la comparaison 208 est la mesure d'une ou de plusieurs valeurs d'écart entre la caractéristique d'acquisition et la caractéristique de consigne.

L'acquisition est poursuivie pendant les étapes de lecture, de calcul et de comparaison. Ces étapes sont toutefois exécutées de manière suffisamment rapide pour que les caractéristiques d'acquisition puissent être considérées comme sensiblement inchangées.

L'étape de comparaison 208 est suivie de deux étapes de décision. Une première étape de décision 210 consiste à déterminer si un temps d'exposition global est atteint ou non. S'il est atteint, l'alimentation de la source est interrompue pour éviter une dose d'exposition trop forte pour le patient et le procédé est terminé en générant une image à partir du signal de lecture de tout ou partie pixels du capteur. L'interruption de l'exposition aux rayons X et la production d'une image à partir des signaux de lecture disponible à cet instant est indiquée par la référence 210 sur la figure 3. Si le temps global d'exposition n'est pas atteint, une deuxième étape de décision 212 a lieu. Elle consiste soit à poursuivre, soit à interrompre l'exposition. L'exposition est poursuivie si l'écart entre la caractéristique d'acquisition et la caractéristique de consigne est trop grand, c'est-à-dire si la caractéristique de consigne n'est pas atteinte. Dans le cas contraire, c'est-à-dire lorsque la caractéristique de consigne est atteinte ou dépassée, on procède à l'étape finale 214. Dans ce cas l'émission des Rayons X est également interrompue et une image est formée avec les signaux de lecture des pixels.

En cas de poursuite de l'exposition, les étapes 202 à 212 sont itérées comme le montre une flèche 216.

L'établissement d'une image radiographique à l'étape 214 peut être suivie, comme le montre une flèche 218 par le rafraîchissement de tous les pixels en vue d'une nouvelle prise de vue.

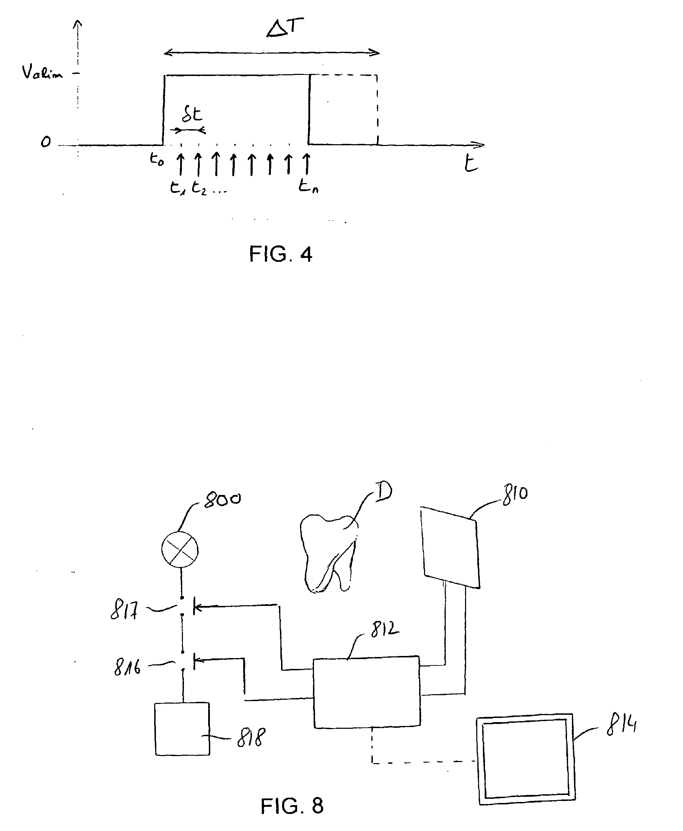

Le chronogramme de la figure 4 permet de mieux illustrer le fonctionnement dans le temps du dispositif. Le chronogramme indique en ordonnée la tension d'alimentation appliquée à la source de rayons X du dispositif de radiographie. On considère que la source n'émet des rayons X que lorsque la tension est égale à la valeur d'alimentation nominale Vₐₗᵢₘ. En abscisse, le chronogramme indique, en échelle libre, le temps.

A un instant t₀, la source est mise sous tension, et le capteur, dont les pixels ont été préalablement rafraîchis, intègre la lumière reçue. La durée maximum de l'exposition, notée ΔT, est fixée en fonction de la dose maximum de rayons X que l'on souhaite administrer à un patient. Elle est de l'ordre de 80 à 200 millisecondes.

Pendant cette durée, à des intervalles réguliers ou irréguliers δt, plus rapprochés, de l'ordre de 10 à 15 millisecondes, on effectue des lectures intermédiaires de tout ou partie des pixels en actionnant les commandes de lecture de la manière déjà décrite. Les lectures intermédiaires sont indiquées par de petites flèches verticales. Elles sont utilisées pour évaluer si la caractéristique d'acquisition de consigne est atteinte ou non. Lorsque cela est le cas, en l'occurrence à l'instant tₙ indiqué sur le figure 4, le signal de lecture des pixels est utilisé pour établir une image de radiologie et la source de rayons X est mise hors tension. On observe ainsi qu'une partie du temps d'exposition a pu être évitée. Cette partie est représentée en trait discontinu à titre indicatif.

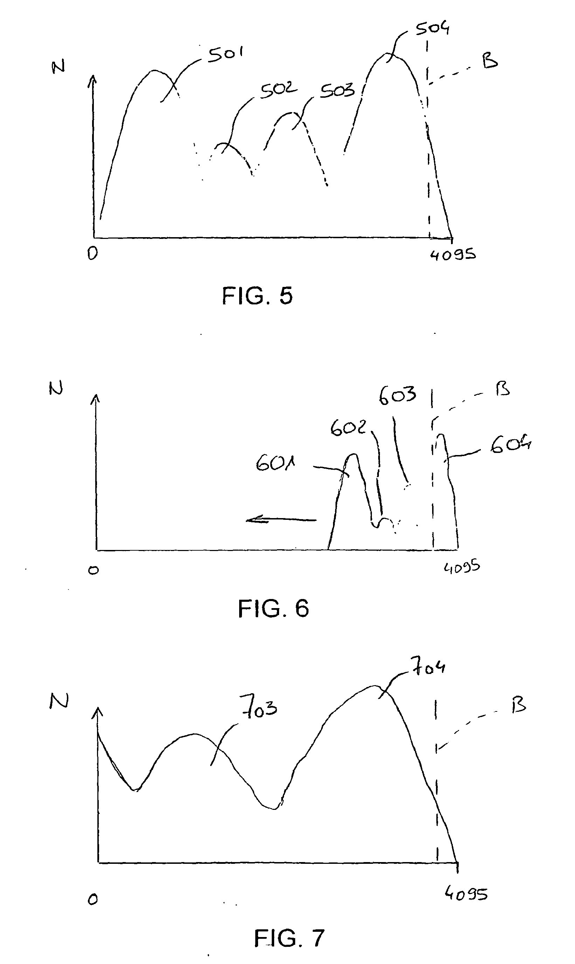

Les figures 5, 6 et 7 illustrent l'utilisation d'une caractéristique d'acquisition d'image particulière, sous la forme d'histogrammes. Cette illustration est donnée pour le cas particulier de la radiographie dentaire.

La figure 5 montre un histogramme type d'une radiographie dentaire correctement exposée, couvrant exactement la latitude d'exposition disponible. L'histogramme indique en abscisse une gamme de valeurs de densité, ou de niveaux de gris, qui d'étend continûment depuis des densités faibles (noir), correspondant aux tissus transparents, dans la partie gauche de la figure, aux densités fortes (blanc) correspondant aux tissus opaques, dans la partie droite de la figure. En ordonnée, l'histogramme indique le nombre de pixels correspondant respectivement à chaque valeur de densité. Le signal du capteur étant converti en un signal numérique, codé en l'occurrence sur 12 bits, les valeurs de densité s'échelonnent entre 0 et 4095. Une ligne B en trait discontinu indique une limite au delà de la le nombre de photons X reçus est faible et où le rapport signal sur bruit est dégradé au point de ne plus distinguer les tissus radiographiés.

On peut observer sur l'histogramme de la figure 5 quatre pics qui correspondent à des densités caractéristique d'une radiographie dentaire. Un premier pic 501, de faible densité correspond à la joue du patient. Un deuxième pic 502, plus dense, correspond à l'os de la mâchoire ou à la dentine des dents. Un troisième pic 503, encore plus dense, correspond à l'émail des dents. Enfin, un quatrième pic 504, quasiment opaque, correspond aux éventuels amalgames présents sur une dent. L'enveloppe, ou l'étalement d'un histogramme comparable à celui de la figure 5 peut être utilisé, par exemple comme caractéristique d'acquisition de consigne.

A titre de comparaison, la figure 6 montre un histogramme établi lors d'une lecture intermédiaire des pixels. La dose reçue est encore faible et les pics de l'histogramme 601, 602, 603, 604, qui correspondent respectivement aux pics 501, 502, 503 et 504 de la figure 5, se trouvent ramassés du coté des fortes densités, c'est-à-dire des matières opaques. Ceci est dû à la faible quantité de lumière intégrée à cet instant. Il s'agit, par exemple de l'instant t1 de la figure 4. Au fur et à mesure que l'exposition aux rayons X est prolongée l'histogramme évolue, les pics prennent de l'ampleur et l'étalement s'accroît dans le sens indiqué par une flèche pour atteindre la forme de la figure 5. A ce moment l'exposition aux rayons X peut être suspendue.

Si l'exposition est prolongée, en revanche, l'histogramme évolue davantage encore et prend une forme telle que représentée à la figure 7. Seuls des pics 703 et 704, correspondant aux pics 503 et 504 de la figure 1 subsistent. Les autres pics ont disparu vers le côté gauche de l'histogramme. Ceci traduit une sur-exposition ou un éblouissement du capteur, c'est à dire une décharge complète du condensateur d'un nombre croissant de ses pixels.

Des caractéristiques d'acquisition de consigne différentes peuvent être retenues en fonction du type d'examen auquel le praticien souhaite procéder. L'histogramme de la figure 5 peut être retenu comme histogramme de consigne pour un examen global de la cavité buccale. L'histogramme de la figure 6, pourrait être retenu pour un examen se limitant à des tissus mous et transparents, tandis que celui de la figure 7 pourrait être retenu comme consigne pour un examen de l'émail ou des amalgames.

La figure 8 représente de manière simplifié un dispositif mettant en oeuvre le procédé décrit. Il comprend ne source de rayons X indiquée avec la référence 800, un capteur de type CMOS indiqué avec la référence 810, une unité de calcul 812, et un moniteur 514. L'unité de calcul 812, par exemple un ordinateur, ou une unité dédiée, a plusieurs fonctions. L'une des fonctions et de recueillir le signal de lecture des pixels du capteur 810 et de fournir au capteur des impulsions de lecture et de rafraîchissement. Elle intègre à cet effet un séquenceur. L'unité de calcul 812 établit également les caractéristiques d'acquisition d'image et les compare à une caractéristique de consigne de la manière déjà décrite. Elle peut aussi utiliser les signaux de lecture pour mettre en forme une image restituée par un moniteur ou un écran 814 qui y est relié.

Enfin l'unité de calcul pilote deux interrupteurs 816 et 817 connectés en série dans un circuit d'alimentation reliant une source d'alimentation électrique 818 à la source de rayons X. Le premier interrupteur 817 est prévu pour interrompre l'émission de rayons X lorsque une durée maximum prédéterminée ΔT est atteinte. Le deuxième interrupteur est prévu pour interrompre l'alimentation lorsque la valeur d'acquisition de consigne est atteinte.

La référence D, représente symboliquement des tissus, en particulier une dent, située dans le champ de radiographie entre la source et le capteur. Le capteur 810 est en particulier un capteur intra-oral.

### Documents cités

1) US 6,856,249
2) US 6,230,975
3) US 2002/0134911
4) EP-B-0858212

## Revendications

1. - Procédé d'asservissement d'une source de rayons X (800) dans un dispositif de radiographie comprenant un capteur d'image (810) du type MOS avec des pixels (10) pourvus respectivement d'une commande de lecture (30) et d'une commande de rafraîchissement (20) indépendantes, **caractérisé en ce que** lors d'une opération de radiographie on sollicite de manière répétée les commandes de lecture d'une pluralité de pixels du capteur d'image tout en alimentant la source de rayons X, de manière a établir, en réponse à chaque lecture au moins une caractéristique d'acquisition d'image, et dans lequel on interrompt l'émission de rayons X lorsque la caractéristique d'acquisition d'image correspond à une caractéristique d'acquisition d'image de consigne.

2. - Procédé selon la revendication 1, dans lequel la caractéristique d'acquisition d'image est un histogramme.

3. - Procédé selon la revendication 2, dans lequel la caractéristique de consigne est une forme d'histogramme, et, en particulier, une forme d'histogramme centrée sur au moins l'un parmi des pics (501, 502, 503, 504, 601, 602, 603, 604, 703, 704),caractéristiques des tissus mous, des os et de la dentine, de l'émail et des matières opaques.

4. - Procédé selon la revendication 1, dans lequel la caractéristique d'acquisition d'image est un nombre de pixels correspondant à au moins une valeur de densité d'image.

5. - Procédé selon la revendication 1 dans lequel la caractéristique d'acquisition d'image est un étalement du spectre de densité dépassant une valeur seuil.

6. - Procédé selon la revendication 1 dans lequel on transmet des commandes de lecture à l'ensemble des pixels du capteur d'image, respectivement à un sous ensemble de pixels du capteur d'image, et on établit la caractéristique d'acquisition d'images à partir de signaux de lecture de tous les pixels, respectivement du sous ensemble de pixels du capteur d'image.

7. - Procédé selon la revendication 6, dans lequel le sous ensemble de pixels comprend des pixels uniformément répartis à la surface du capteur.

8. - Procédé selon la revendication 1 dans lequel on sollicite les commandes de lecture de manière séquentielle.

9. - Procédé de radiographie utilisant le procédé d'asservissement de la revendication 1 dans lequel on effectue une lecture des pixels du capteur lorsque la caractéristique d'acquisition d'image correspond à une caractéristique d'acquisition d'image de consigne, et on forme une image à partir d'un signal de lecture obtenu lors de la lecture.

10. - Dispositif de radiographie comprenant une source de rayons X (806) et un capteur d'image (810) du type MOS avec des pixels pourvus respectivement d'une commande de lecture et d'une commande de rafraîchissement indépendantes, un séquenceur pour appliquer aux commandes de lecture une série de signaux de sollicitation, répartis sur une période d'exposition radiologique, un calculateur (812) recevant des signaux de lecture fournis par les pixels en réponse aux signaux de sollicitation pour établir une caractéristique d'acquisition d'image et pour comparer la caractéristique d'acquisition à une caractéristique d'acquisition de consigne, et **caractérisé en ce qu**'il comporte un moyen (817) d'interruption de l'exposition radiologique, piloté par le calculateur, pour interrompre l'exposition lorsque la caractéristique d'acquisition correspond à la caractéristique d'acquisition d'image de consigne.

11. - Dispositif selon la revendication 10, comprenant en outre un moyen (814) de restitution d'image pour fournir une image à partir des signaux de lecture fournis par les pixels.

## Claims

1. A method of controlling an X-ray source (800) in a radiography device comprising an image sensor (S10) of MOS type with pixels (10) respectively provided with a read command (30) and a refresh command (20) which are independent, **characterised in that**,in a radiography operation the read commands of a plurality of pixels of the image sensor are repeatedly actuated while powering the X-ray source in such a way as to establish in response to each reading at least one image acquisition characteristic, and in which the emission of X-rays is interrupted when the image acquisition characteristic corresponds to a reference image acquisition characteristic.

2. A method according to claim 1 wherein the image acquisition characteristic is a histogram.

3. A method according to claim 2 wherein the reference characteristic is a form of histogram and in particular a form of histogram centered on at least one among peaks (501, 502, 503, 504, 601, 602, 603, 604, 703, 704) which are characteristic of soft tissues, bones and dentine, enamel and opaque materials.

4. A method according to claim 1 wherein the image acquisition characteristic is a number of pixels corresponding to at least one image density value.

5. A method according to claim 1 wherein the image acquisition characteristic is a spread of the density spectrum exceeding a threshold value.

6. A method according to claim 1 wherein read commands are transmitted to all of the pixels of the image sensor or respectively to a sub-assembly of pixels of the image sensor and the image acquisition characteristic is established from reading signals of all the pixels or respectively the sub-assembly of pixels of the image sensor.

7. A method according to claim 6 wherein the sub-assembly of pixels comprises pixels uniformly distributed at the surface of the sensor.

8. A method, according to claim 1 wherein the read commands are actuated sequentially.

9. A radiography method using the control method of claim 1 wherein reading of the pixels of the sensor is effected when the image acquisition characteristic corresponds to a reference image acquisition characteristic and an image is formed from a read signal obtained in the reading operation.

10. A radiography device comprising an X-ray source (800) and an image sensor (810) of MOS type with pixels respectively provided with a read command and a refresh command which are independent, a sequencer for applying to the read commands a series of actuation signals distributed over a radiological exposure period, a calculating means (812) receiving read signals provided by the pixels in response to the actuating signals to establish an image acquisition characteristic and to compare the acquisition characteristic to a reference acquisition characteristic, **characterised in that** it comprises a means (817) for interruption of the radiological exposure, which is driven by the calculating means, to interrupt the exposure when the acquisition characteristic corresponds to the reference image acquisition characteristic.

11. A device according to claim 10 further comprising an image restitution means (814) for providing an image from the read signals provided by the pixels.

## Patentansprüche

1. Verfahren zum Regeln einer Röntgenstrahlenquelle (800) in einer Röntgenvorrichtung, die einen Bildaufnehmer (810) des MOS-Typs mit Pixeln (10) umfasst, die mit einer Lesesteuerung (30) und mit einer Auffrischungssteuerung (20), die voneinander unabhängig sind, versehen sind, **dadurch gekennzeichnet, dass** bei einem Röntgenbetrieb wiederholt die Lesesteuerungen mehrerer Pixel des Bildaufnehmers stimuliert werden und dabei die Röntgenstrahlenquelle gespeist wird, derart, dass in Reaktion auf jedes Lesen wenigstens ein Bilderfassungsmerkmal gebildet wird, wobei die Aussendung von Röntgenstrahlen unterbrochen wird, wenn das Bilderfassungsmerkmal einem Schwellen-Bilderfassungsmerkmal entspricht.

2. Verfahren nach Anspruch 1, wobei das Bilderfassungsmerkmal ein Histogramm ist.

3. Verfahren nach Anspruch 2, wobei das Schwellenmerkmal eine Histogrammform und insbesondere eine auf wenigstens einen von mehreren Peaks (501, 502, 503, 504, 601', 602, 603, 604, 703, 704) zentrierte Histogrammform ist, wobei die Peaks für Weichgewebe, Knochen und Zahnbein, für Emaille bzw. lichtundurchlässige Substanzen charakteristisch sind.

4. Verfahren nach Anspruch 1, wobei das Bilderfassungsmerkmal eine Anzahl von Pixeln ist, die wenigstens einem Dichtewert des Bildes entspricht.

5. Verfahren nach Anspruch 1, wobei das Bilderfassungsmerkmal eine Verbreiterung des Dichtespektrums, die einen Schwellenwert übersteigt, ist.

6. Verfahren nach Anspruch 1, wobei Lesebefehle an sämtliche Pixel des Bildaufnehmers bzw. an eine Untergesamtheit von Pixeln des Bildaufnehmers gesendet werden und das Bilderfassungsmerkmal anhand von Lesesignalen für sämtliche Pixel bzw. für die Untergesamtheit von Pixeln des Bildaufnehmers gebildet wird.

7. Verfahren nach Anspruch 6, wobei die Untergesamtheit von Pixeln Pixel umfasst, die auf der Oberfläche des Aufnehmers gleichmäßig verteilt sind.

8. Verfahren nach Anspruch 1, wobei die Lesesteuerungen sequentiell stimuliert werden.

9. Röntgenverfahren, das das Regelungsverfahren nach Anspruch 1 verwendet, wobei ein Lesen von Pixeln des Aufnehmers ausgeführt wird, wenn das Bilderfassungsmerkmal einem Schwellen-Bilderfassungsmerkmal entspricht, und ein Bild anhand eines beim Lesen erhaltenen Lesesignals erzeugt wird.

10. Röntgenvorrichtung, die eine Röntgenstrahlenquelle (806) und einen Bildaufnehmer (810) des MOS-Typs mit Pixeln, die mit einer Lesesteuerung bzw. mit einer Auffrischungssteuerung, die voneinander unabhängig sind, versehen sind, eine Ablaufsteuerung, um in die Lesesteuerungen eine Reihe von Stimulationssignalen einzugeben, die auf eine Röntgenbestrahlungsperiode verteilt sind, und einen Rechner (812), der die von den Pixeln in Reaktion auf die Stimulationssignale gelieferten Lesesignale empfängt, um ein Bilderfassungsmerkmal zu schaffen und um das Erfassungsmerkmal mit einem Schwellen-Erfassungsmerkmal zu vergleichen, umfasst, **dadurch gekennzeichnet, dass** sie ein Mittel (817) zum Unterbrechen der Röntgenbestrahlung umfasst, die durch den Rechner gesteuert wird, um die Bestrahlung zu unterbrechen, wenn das Erfassungsmerkmal dem Schwellen-Bilderfassungsmerkmal entspricht.

11. Vorrichtung nach Anspruch 10, die außerdem ein Mittel (814) zum Wiederherstellen eines Bildes umfasst, um ein Bild anhand von Lesesignalen, die von den Pixeln geliefert werden, zu erzeugen.
